# EUROPEAN PATENT APPLICATION

(11) **EP 0 855 193 A1**
(43) Date of publication of application: **29.07.1998**
(21) Application number: 97500225.4
(22) Date of filing: 29.12.1997
(51) Int. Cl.: A61M 3/02

(54) **Intestinal cleansing device**

(30) Priority: 23.01.1997 ES 9700149
(71) Applicant: Mendoza Trevilla, Angel, 48950 Astrabudua-Erandio Vizcaya (ES); Aretxabaleta Ayo, Maria Jose, 48950 Astrabudua-Erandio Vizcaya (ES)
(72) Inventor: Mendoza Trevilla, Angel, 48950 Astrabudua-Erandio Vizcaya (ES); Aretxabaleta Ayo, Maria Jose, 48950 Astrabudua-Erandio Vizcaya (ES)
(74) Representative: Perez Bonal, Bernardo

(57) **Abstract**

It is applicable in the anus of the user in order to solve problems of constipation by the introduction of generally warm water directed towards the intestine.

It is constituted by a cannula (1) configured by a cylindrical front section (1.1) of rounded edges, from which a bell shaped extension (1.2) progressively extends from its rear part, ending in a vertical face, from which in turn perpendicularly starts a threaded stem (1.3) on which a flexible tube (2) is adjusted, capable of being coupled to a tap on its other end, the cannula (1) likewise being provided with a through hole (1.4) through which longitudinally circulates the water coming from the flexible tube (2).

## Description

### OBJECT OF THE INVENTION

The present invention refers to an intestinal cleansing apparatus, the purpose of which is to reduce intestinal discomforts caused by a situation of constipation, also intending to minimize the haemorrhoid afflictions derived from the added effort the person subjected to these circumstances must exert when trying to defecate.

An object of the invention is to provide an apparatus which is easily adaptable to the anus of the user by means of a cannula with optimized configuration for comfortable application, which, at the same time shall be equipped with adjustment means for the incorporation of a flexible conduit through which warm water flows, generally coming from the supply system, and which shall penetrate through the cannula, directed towards the intestine of the user.

### BACKGROUND OF THE INVENTION

The problems caused by constipation result to be considerable, the longer this situation lasts, being specially uncomfortable in specific intestinal areas in which an excessive accumulation of faeces is produced, causing an over pressure of the intestinal circulation ducts, producing pain.

This situation becomes specially aggravated in those persons who suffer haemorrhoidal problems, since under said circumstances of constipation, the effort the person affected must make during his intent to evacuate the faeces, is considerable, and may provoke a bursting of the haemorrhoids, causing great pain.

Diverse solutions exist for constipation, enemas being the most extensively known apparatus for their application on the anus of the user.

The enema is provided with a cannula which is placed so that it remains incorporated inside the anus, facilitating the introduction of generally warm water, which has been previously accumulated in a receptacle on which the user exerts pressure to enable its exit.

This type of apparatus, requires the user to act in alternate manner, pressing on the receptacle and consecutively leaving it expand, with the object of achieving a pumping of the liquid it contains inside, so as to direct it towards its exit.

On the other hand, operational limitations of the enema exist, determined by the actual capacity of the receptacle, which on occasions results to be very reduced for its needs, making it necessary to fill the receptacle with liquid for re-use.

Said negative circumstances associated to the actual operationability of the enema makes the development of another type of apparatus which permits the user to have a greater facility of use, feasible, this being the object of the invention which is herewith described.

### DESCRIPTION OF THE INVENTION

The intestinal cleansing apparatus, which is the object of this invention, covers the indicated expectations to a total satisfaction, providing a simple means of application, of easy use, which permits the continuous circulation of fluid for conducting the intestinal cleansing.

The intestinal cleansing apparatus is essentially constituted by, a cannula manually adapted to the anus of the user and by a flexible tube, coupled at its rear part to the cannula and which in turn is found linked on its other end to a tap of a sanitary equipment from which warm water generally flows, facilitating in this manner, that the water be directed towards the inside of the body of the user, in order to solve the intestinal ills which harass his health

The continuity of the application of water onto the intestine makes possible a softening of the accumulated faeces, contributing to a better disintegration, in order to achieve its ideal fluidity during its movement, solving the circulation problems which previously caused the suffering of the user.

The cannula presents an essentially cylindrical configuration with rounded edges on its front section, which constitutes the section which is introduced on the anus, this section extending on its rear part into a bell shaped extension of progressively growing size, of ergonomic configuration, which extends towards a vertical face, from which a central, threaded stem of smaller diameter, perpendicularly derives, on which the flexible tube is threaded, which is associated on its other end to the tap of the sanitary equipment.

The cannula is provided in its interior with a through hole, through which the fluid coming from the flexible tube, circulates, to be subsequently directed towards the intestinal ducts.

The vertical face of the so called bell shaped extension, shall constitute an attachment and supporting section for the fingers of the user, who, by means of a simple pressure on the cannula, shall ensure its adjustment during application.

Additionally, and in a satisfactory manner, the possibility of conferring other uses to the described cleansing apparatus is contemplated, for example it may result to be applicable for vaginal washing

### DESCRIPTION OF THE DRAWINGS

In order to complement this description and with the purpose of helping to a better understanding of the characteristics of the invention, the present Specification is enclosed with a set of drawings, forming integral part of the same, in which, with illustrative and non limitative character, a side view of the intestinal cleansing apparatus has been represented, showing the input and output direction of the fluid.

### PREFERRED EMBODIMENT OF THE INVENTION

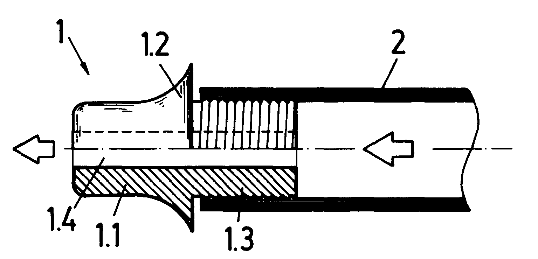

In view of the figure, it may be seen that the intestinal cleansing apparatus, which is the object of this invention, and which is applicable on the anus of the user, is of the type which is used to solve problems of constipation by the insertion of generally warm water inside the intestine of the user.

The intestinal cleansing apparatus is essentially found to be constituted by a cannula (1) configured by a cylindrical front section (1.1) of rounded edges, from which a bell shaped extension (1.2) projects progressively from the rear part, ending in a vertical face, from which in turn, a threaded stem (1.3) starts perpendicularly, and on which a flexible tube (2) is attached, capable of being coupled to a tap by its other end, the cannula (1) likewise being provided with a through hole (1.4), through which longitudinally, circulates the water from the flexible tube (2).

A more extensive description is not considered necessary to make any expert in the art understand the scope of the invention and the advantages derived from the same.

The materials, shape, size and arrangement of the elements shall be capable of variation provided they do not alter the essentiality of the invention.

The terms in which this Specification has been described, shall always be taken in their wide and non limitative meaning.

## Claims

1. Intestinal cleansing apparatus of the type which is applicable on the anus of the user in order to solve problems of constipation by the introduction of generally warm water directed towards the intestine, essentially characterized in that it is constituted by a cannula (1), configured by a cylindrical front section (1.1) of rounded edges from which a bell shaped extension (1.2) progressively extends from its rear part, ending in a vertical face, from which in turn, a threaded stem (1.3) starts perpendicularly, on which a flexible tube (2) is adjusted, capable of being coupled to a tap by its other end, the cannula (1) likewise being provided with a through hole (1.4) through which longitudinally, the water coming from the flexible tube (2) circulates.
